# EUROPEAN PATENT APPLICATION

(11) **EP 2 107 061 A1**
(43) Date of publication of application: **07.10.2009**
(21) Application number: 08153990.0
(22) Date of filing: 02.04.2008
(51) Int. Cl.: C07D 495/04

(54) **Process for the preparation of optically enriched clopidogrel**

(71) Applicant: Krka Tovarna Zdravil, D.D., Novo Mesto, 8501 Novo Mesto (SI)
(72) Inventor: Veverkovà, Eva, SK-847 08 Bratislava (SK); Veverka, Miroslav, SK-847 08 Bratislava (SK); Simonic, Igor, SLO-8351 Straza (SI); Smodis, Janez, SLO-8000 Novo mesto (SI); Zupet, Rok, SLO-1211 Ljubljana (SI)
(74) Representative: UEXKÜLL & STOLBERG

(57) **Abstract**

The invention relates to a process for the preparation of substantially optically pure or optically enriched clopidogrel or a pharmaceutically acceptable salt or solvate thereof. The invention also relates to a process for the preparation of a pharmaceutical formulation comprising substantially optically pure clopidogrel, or a pharmaceutically acceptable salt or solvate thereof, in combination with at least one pharmaceutically acceptable carrier.

## Description

The invention relates to a process for the preparation of optically enriched and preferably optically pure clopidogrel and the pharmaceutically acceptable salts and solvates thereof by optical resolution.

### Background of the invention

Clopidogrel is the INN name for (S)-(+)-methyl 2-(2-chlorophenyl)-2-(6,7-dihydrothieno[3,2-c]pyridin-5(4H)-yl) acetate:

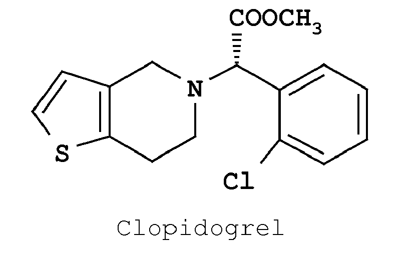

This compound is the (S)-isomer of methyl 2-(2-chlorophenyl)-2-(6,7-dihydrothieno[3,2-c]pyridin-5(4H)-yl)acetate which is represented by formula (I):

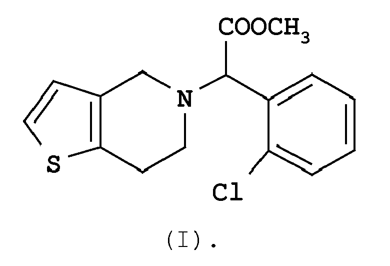

Clopidogrel is a known antiplatelet agent, which works by preventing platelets from sticking together to form clots that would restrict blood flow. Clopidogrel is particularly used for inhibiting platelet aggregation or decreasing the frequency of atherosclerotic events (brain strokes or cardiac attacks, mortality due to blood vessel disease) in patients with a symptomatic form of atherosclerotic disease such as recovered brain stroke, myocardial infarct and peripheral arterial occlusive disease. The drug is currently being marketed in the form of clopidogrel bisulfate salt. A particular dosage form contains an amount of clopidogrel bisulfate which is the equivalent of 75 mg of clopidogrel base.

Clopidogrel is generally disclosed in US 4,529,596, US 6,258,961, US 5,036,156, US 6,080,875, US 6,180,793 and FR 2769313.

US 4,847,265 describes a process for the preparation of the (+)-isomer of the compound of formula (I) by salt formation from racemic compound of formula (I) and an optically active acid such as (-)-camphor-10-sulfonic acid, crystallization from a solvent such as acetone and subsequent liberation of the (+)-isomer of the compound of formula (I) using a base.

US 6,737,411 describes a process for the separation of enantiomers of the compound of formula (I) by crystallization as camphorsulfonate salt from a mixture of a hydrocarbon and a co-solvent such as DMF, butanol or acetone.

WO 02/059128 describes the optical resolution of inter alia the compound of formula (I) by formation of diastereomeric salt and crystallizing from a suitable solvent.

WO 2004/074215 describes a process for optical resolution of enantiomers of the compound of formula (I) by crystallization of a camphor sulfonate salt thereof from a solvent. Preferred solvents are specific bis(alkoxyethyl) ethers.

US 2005/0059696 describes a process of optical resolution using an acid addition salt with a chiral acid such as tartaric acid, mandelic acid, lactic acid, camphorsulfonic acid and amino acids and a solvent such as alcohol, ketone, ethyl acetate, methyl acetate, DMF, acetonitrile, propionitrile, THF and dioxane.

US 2005/049275 discloses a processes for the separation of enantiomers of the compound of formula (I) by crystallization as camphor sulfonate salt from a mixture of a hydrocarbon and a co-solvent such as DMF, butanol or acetone.

WO 2005/077958 discloses a process for the resolution of the enantiomers of the compound of formula (I) by diastereomeric crystallization comprising the use of a (S)-camphorsulfonic acid and at least one solvent. Preferred solvents are ketones and toluene.

WO 2006/094468 discloses racemization of the compound of formula (I) using quaternary ammonium hydroxide as well as resolution of the enantiomers of the compound of formula (I) by crystallization from a mixture of an ester such as isopropyl acetate and an alcohol such as methanol.

Chiral solvents are reported to have been already used as a sole inducer of enantiomeric excess in organic reactions. Some chiral solvent induction methods for enantioseparation or synthesis were published (Helv. Chim. Acta 1979, 62, 2695; A. Chimia 1983, 37, 449; Tetrahedron Asymmetry 2003, 3081; Tetrahedron Lett. 2004, 6425; Org. Lett. 2005, 7, 335; Tetrahedron 2006, 12420; Advanced Organic Chemistry, Wiley-Interscience 2001, 150; US 6,486,355).

WO 99/65915 discloses polymorphs I and II of clopidogrel hydrogen sulfate, WO 03/51362 discloses polymorphs III, IV, V and VI and an amorphous form as well as a process for the preparation of polymorphs I and II.

The amorphous clopidogrel hydrogen sulfate and its preparation are described in WO 00/010534, WO 04/26879, WO 04/81015, WO 04/81016, WO 04/98593 and WO 05/16931.

WO 03/66637 discloses polymorphs I and II of clopidogrel hydrochloride and WO 05/117866 discloses an amorphous form of clopidogrel hydrochloride. WO 07/029096 discloses polymorph form III of clopidogrel hydrochloride. WO 07/029080, discloses the preparation of form I of clopidogrel hydrochloride from various ethers.

The crystallization of polymorphs of clopidogrel salts is an object of numerous patent applications disclosing also the synthesis of clopidogrel: WO 04/20443, WO 04/48385, WO 04/52966, WO 04/106344, WO 05/012300, WO 05/16931, WO 05/26174, WO 05/63708, WO 05/68471, WO 05/80890, WO 05/97804, WO 05/100364, WO 05/103059, WO 05/104663, WO 06/23676, WO 06/34451, WO 06/87226 and WO 06/87729.

Formulations of clopidogrel and its salts as well as processes for its preparation are described in WO 04/74215, WO 04/72085, WO 04/72084, WO 05/48992, WO 05/70464, WO 06/44548, WO 06/74066, WO 06/91847.

### Description of the invention

It is an object of the present invention to provide a process for preparing optically enriched and preferably substantially optically pure clopidogrel which process can advantageously be applied on an industrial scale.

It has surprisingly been found that this object can be achieved by a process for the preparation of optically enriched clopidogrel of the following formula 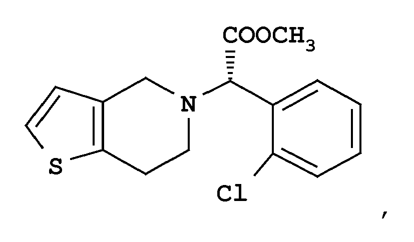 or a pharmaceutically acceptable salt or solvate thereof, comprising the steps of:
a) contacting a mixture comprising both enantiomers of the compound of formula (I) 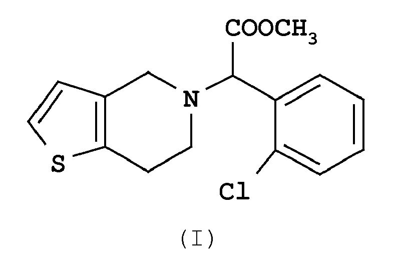 or a salt thereof with an optically active acid in a solvent or solvent mixture to give a mixture of diastereomeric acid addition salts,
   wherein the solvent or solvent mixture comprises at least one solvent selected from the group consisting of optically active solvents, linear or branched (C₁-C₅)alkoxy(C₂-C₅) alcohols and linear or branched acyclic (C₁-C₅)alkoxy(C₂-C₅)alkyl (C₁-C₅)alkyl ethers;
b) separating the diasteromeric acid addition salts;
c) optionally reacting at least one of the separated acid addition salts with a base to give optically enriched clopidogrel; and
d) optionally converting the optically enriched clopidogrel into a pharmaceutically acceptable salt or solvate thereof.

A "substantially optically pure" isomer in the context of the present invention generally means an isomer with a diastereomeric excess (d.e.) or enantiomeric excess (e.e.) acceptable for a chiral compound prepared on industrial scale. Such d.e. and e.e. values are readily determined by a person skilled in the art. For example, determination of enantiomeric purity may be carried out by proton NMR spectroscopy with the addition of a chiral rare earth reagents (shift reagents), by HPLC using a chiral stationary phase or by measurement of optical rotation. Usually, a process is suitable for preparation on industrial scale with an d.e. or e.e. of at least 85 %, preferably of at least 90 % and more preferably of at least 95 %.

The process according to the present invention using a specific solvent system has a number of advantages. In particular, the inventive process may be carried out using commercially viable solvents, such as commercially viable chiral solvents. Moreover, in the case of using a multicomponent solvent system, the individual solvents can be easily recovered and recycled using fractional distillation processes generally known in the art.

Most importantly, the process according to the invention allows to efficiently provide clopidogrel as well as pharmaceutically acceptable salts and solvates thereof straightforwardly after a single crystallization in an enantiomeric purity of >> 99 %, such as an enantiomeric purity of at least 99.7 %. Subsequently, the product thus obtained may be re-crystallized in order to prepare clopidogrel and pharmaceutically acceptable salts and solvates thereof having an enantiomeric purity of ≥ 99.8 %.

In step (a), a mixture comprising both enantiomers of the compound of formula (I) or a salt thereof is contacted with an optically active acid. In said mixture the enantiomers of the compound of formula (I) may be present in equimolar amounts, or the mixture may be enriched in one of the enantiomers. A mixture of enantiomers of the compound of formula (I) may be obtained according to processes generally known in the art. Alternatively, mixtures of the enantiomers of the compound of formula (I) which are commercially available can be used in the process according to the invention.

Generally, step (a) can be performed in a single solvent or a solvent mixture.

According to one embodiment, the solvent or solvent mixture of step (a) comprises at least one optically active solvent.

According to another embodiment, the solvent or solvent mixture of step (a) comprises at least one linear or branched (C₁-C₅)alkoxy(C₂-C₅) alcohol.

According to another embodiment, the solvent or solvent mixture of step (a) comprises at least one linear or branched acyclic (C₁-C₅)alkoxy(C₂-C₅)alkyl (C₁-C₅)alkyl ether.

According to another embodiment, the solvent mixture of step (a) comprises at least one optically active solvent in combination with at least one linear or branched (C₁-C₅)alkoxy(C₂-C₅) alcohol.

According to another embodiment, the solvent mixture of step (a) comprises at least one optically active solvent in combination with at least one linear or branched acyclic alkoxy ether selected from (C₁-C₅)alkoxy(C₂-C₅)alkyl (C₁-C₅)alkyl ethers and bis ((C₁-C₅) alkoxy (C₂-C₅)alkyl) ethers, wherein (C₁-C₅)alkoxy(C₂-C₅)alkyl (C₁-C₅)alkyl ethers are preferred.

According to another embodiment, the solvent mixture of step (a) comprises at least one linear or branched (C₁-C₅)alkoxy(C₂-C₅)alcohol in combination with at least one linear or branched acyclic alkoxy ether selected from (C₁-C₅)alkoxy(C₂-C₅)alkyl (C₁-C₅)alkyl ethers and bis (C₁-C₅)alkoxy(C₂-C₅)alkyl) ethers, wherein (C₁-C₅)alkoxy(C₂-C₅)alkyl (C₁-C₅)alkyl ethers are preferred.

Examples of optically active solvents include esters of optically active acids with linear or branched (C₁-C₅)alcohols. Suitable optically active acids include tartaric acid, malic acid, 3-hydroxybutyric acid and lactic acid, more preferably lactic acid. Particularly preferred optically active solvents include esters of L-lactic acid with a linear or branched (C₁-C₅)alcohol, such as methyl L-lactate or ethyl L-lactate.

Examples of linear or branched (C₁-C₅)alkoxy(C₂-C₅)alcohols include ethoxyethanol and isopropoxyethanol.

Examples of (C₁-C₅)alkoxy(C₂-C₅)alkyl (C₁-C₅)alkyl ethers include methoxyethyl methyl ether (glyme).

Examples of bis((C₁-C₅)alkoxy(C₂-C₅)alkyl) ethers include bis (2-methyoxyethyl) ether (diglyme).

In addition to the solvents and solvent combiantions defined in any of the above embodiments, the solvent mixture of step (a) can comprise additional solvents. Preferably, the solvent mixture comprises at least one additional solvent selected from the group consisting of linear or branched aliphatic (C₁-C₆)alcohols, cyclic or acyclic aliphatic (C₁-C₆)ethers, aliphatic (C₂-C₈)esters, aliphatic (C₂-C₆)ketones and (C₅-C₁₂)hydrocarbons. Esters, ethers or ketones are particularly preferred.

Examples of linear or branched aliphatic (C₁-C₆)alcohols include methanol, ethanol, propanol, isopropanol and butanol. Butanol and ethanol are particularly preferred.

Examples of cyclic or acyclic aliphatic (C₁-C₆)ethers include tert-butyl methyl ether and tetrahydrofuran.

Examples of aliphatic (C₂-C₈)esters include butyl acetate and methyl acetate.

Examples of aliphatic (C₂-C₆)ketones include acetone and methylethylketone.

Examples of (C₅-C₁₂)hydrocarbons include toluene, xylene, benzene and chlorobenzene. Toluene is particularly preferred.

According to a particularly preferred embodiment, the solvent mixture used in step (a) comprises at least one chiral solvent in combination with at least one additional solvent selected from the group consisting of butanol, ethanol, ethoxyethanol, isopropoxyethanol, glyme, diglyme, n-butyl acetate, methyl acetate and toluene.

According to another preferred embodiment, the solvent mixture used in step (a) comprises at least one solvent selected from the group consisting of ethoxyethanol, isopropoxyethanol and glyme in combination with at least one additional solvent selected from the group consisting of n-butyl acetate and methyl acetate.

The solvent mixture used in step (a) may optionally comprise water. According to a preferred embodiment, the solvent mixture comprises 0 % - 5 % (v/v), particularly 0.5 - 2 % (v/v) of water.

Is has been found that the amount of chiral solvent, such as e.g. ethyl L-lactate, influences the degree of optical resolution and thus the diastereomeric excess obtained. Particularly, increasing the amount of chiral solvent may improve the degree of optical resolution.

Preferably, the solvent mixture used in step (a) comprises optically active solvent in an amount of up to 50 % (v/v), preferably 2 - 30 % (v/v), most preferably 5 - 25 % (v/v) based on the total volume of solvents. It is further preferred that the weight ratio of the optically active solvent to the compound of formula (I) is in the range of 0.5:1 to 50:1.

Suitable optically active acids for use in step (a) generally include the chiral acids commonly used for diastereomeric salt formation. Examples of suitable optically active acids include (1R)-(-)- or (1S)-(+)-camphor-10-sulfonic acid, (+)- or (-)-tartaric acid, L-(-)-0,0'-dibenzoyl tartaric acid, L-(+)-ascorbic acid, L-(+)-asparaginic acid, L-(+)-N-acetyl aspartic acid, L-(+)-aspartic acid, L-(+)-asparaginic acid, L-(+)-N-acetylasparaginic acid, L-(+)-glutamic acid, L-(+)-N-acetylglutamic acid or (S)-(-)-2-[(phenylamino)carbonyloxy]-propionic acid. Preferably, the optically active acid is selected from the group consisting of tartaric acid, mandelic acid, lactic acid, camphorsulfonic acid and bromocamphorsulfonic acid. (R)-camphor-10-sulfonic acid is particularly preferred. It is further preferred that the optically active acid is used in an amount of 0.4 to 1.2 equivalents with respect to the compound of formula (I).

Step (a) may generally be performed at any suitable temperature, such as at a temperature from 0 °C to the reflux temperature of the solvent or solvent mixture. More preferably, step (a) is performed at a temperature in the range of 40 °C to 80 °C, even more preferably in the range of 70 °C to 80 °C.

The separation of diastereomeric acid addition salts of the compound of formula (I) in step (b) is typically performed by crystallization from the reaction mixture obtained in step (a). Optionally, one or more additional solvents can be added to the mixture. Subsequently, the resulting mixture is allowed to cool and/or is cooled. For example, the mixture may be allowed to cool down to room temperature and may then be further cooled to a temperature of 0 °C to 10 °C for further crystallization. Finally, crystals are recovered by filtration, optionally washed with a solvent and dried.

Generally, crystallization may occur spontaneously or may be effected by addition of one or more additional solvents, by salting out or by partial evaporation of solvent. Moreover, crystallization may further comprise seeding the mixture with the desired acid addition salt. Preferably, seeding is performed during the cooling process of the mixture, such as at a temperature of 50 °C to 70 °C. Alternatively, crystallization may be effected simply by stirring the mixture at a suitable temperature.

The acid addition salt obtained by crystallization in step (b) may optionally be further purified by re-crystallization, for example by repeating the crystallization according to step (b) at least once, such as one, twice, three times etc. Thus, the crystallization may be repeated until no further change is observed in the optical rotation of the obtained acid addition salt. While a single crystallization step in accordance with the process of the invention typically already results in a diastereomeric purity of at least 99 % d.e., a further rise in optical purity can be effected by re-crystallization, which allows to reproducibly obtain the acid addition salt of the compound of formula (I) in an optical purity of >> 99 % d.e.

The separation of diastereomeric acid addition salts may alternatively or additionally comprise additional separation steps using methods generally known in the art, such as crystallization from other solvents or column chromatography.

The mother liquor obtained from the crystallization is typically concentrated to a suitable volume and the residue recycled, i.e. used in another optical resolution. More preferably, from the residue obtained from the mother liquor the free base of the compound of formula (I) is recovered and subsequently racemized prior to being used in a further process of optical resolution. Typically, recovery of the free base is performed by reaction with a base as described below for step (c) and racemization is performed under either basic or acidic conditions. Preferably, racemization is effected using an acid, such as acetic acid, formic acid or propionic acid, or a base, such as an organic amine, an alkoxide, e.g. sodium or potassium methoxide, ethoxide or tert-butoxide, or an inorganic base, as generally known in the art. Racemization can be carried out in solutions of the free base of the compound of formula (I) in mixtures of aqueous and organic solvents or organic solvents alone. However, organic solvents alone are preferred due to their higher volatility. Alternatively, racemization can be carried out by reacting the free base of the compound of formula (I) with a liquid acid, such as glacial acetic acid, formic acid or propionic acid, without any solvent. If the racemization results in hydrolysis of the ester group of the compound of formula (I), re-esterification is performed, such as by use of a methylating agent. However, racemization conditions which substantially prevent ester hydrolysis as generally known in the art are preferred.

Thus, step (b) may preferably further comprise at least one cycle of:
(i) recovering the compound of formula (I) from the mother liquor of the crystallization
(ii) racemizing the compound of formula (I) thus obtained
(iii) contacting the racemized compound of formula (I) with an optically active acid to give a diastereomeric mixture of acid addition salts of the compound of formula (I)
(iv) separating the acid addition salts of the compound of formula (I) by crystallization.

The steps of recovery, racemization and resolution may be cycled to obtain a substantially optically pure acid addition salt of clopidogrel in an overall yield of 85 % or more.

According to optimal step (c) of the process according to the invention, at least one of the separated acid addition salts of the compound of formula (I) is optionally reacted with a base to give substantially optically pure or optically enriched clopidogrel, i.e. the (S)-isomer of the compound of formula (I). Typically, the acid addition salt of the (S)-isomer of the compound of formula (I) is dissolved or suspended in a mixture of water and a non-water miscible organic solvent, such as methylene chloride, ethyl acetate, isopropyl acetate or methyl isobutyl ketone, and is neutralized with a base under stirring to liberate clopidogrel as free base. Suitable bases include alkaline and alkaline earth metal bicarbonates and carbonates, such as NaHCO₃, K₂CO₃ or Na₂CO₃. The free base of the compound of formula (I) is recovered from the organic phase by way of separation of the layers and evaporation of the organic solvent.

According to optimal step (d) of the process according to the invention, the substantially optically pure or optically enriched clopidogrel obtained in step (c) may be further transformed into a pharmaceutically acceptable salt or solvate thereof. Examples of pharmaceutically acceptable salts are the hydrogensulfate, sulfate, hydrochloride, hydrobromide, (1R)-(-)-camphor-10-sulfonate and (1S)-(+)-camphor-10-sulfonate. Further examples of pharmaceutically acceptable salts of clopidogrel and polymorphic forms thereof are described in WO 2008/034912. Preferably, the pharmaceutically acceptable salt is clopidogrel hydrogensulfate, even more preferably clopidogrel hydrogensulfate of crystalline form I.

The present invention also relates to a process for the preparation of a pharmaceutical formulation comprising substantially optically pure clopidogrel, or a pharmaceutically acceptable salt or solvate thereof, in combination with at least one pharmaceutically acceptable carrier, which includes the step of preparing the substantially optically pure clopidogrel or the pharmaceutically acceptable salt or solvate thereof according to the process of the invention. Preferably, the pharmaceutically acceptable salt is clopidogrel hydrogensulfate, even more preferably clopidogrel hydrogensulfate of crystalline form I. Further examples of pharmaceutical formulations are described in WO 2008/034912.

The present invention will further be illustrated by means of the following examples.

### Examples

### Example 1

### Camphorsulfonate of the compound of formula (I)

5.0 g of a racemic mixture of the compound of formula (I) were dissolved in 5 mL of (-)-ethyl L-lactate and 2.5 mL butanol at a temperature of 70 °C. 2.2 g of solid (-)-(R)-camphor-10-sulfonic acid were added into the solution. 15 mL of diglyme were further added into the reaction mixture. The mixture was inoculated with 0.1 g of clopidogrel (R)-camphorsulfonate and allowed to cool down to room temperature. The mixture was further stirred at room temperature until crystals began to form and then cooled in a refrigerator. Crystals were recovered by filtration through sintered glass and washed with diethyl ether to obtain 3.1 g of the camphorsulfonate of the compound of formula (I), [α]_{D} = +23.99° (MeOH), having an optical purity of 99.7 % (+)-isomer (determined by chiral HPLC).

### Example 2

### Camphorsulfonate of the compound of formula (I)

33.1 g of a racemic mixture of the compound of formula (I) were dissolved in 264 mL of a mixture of (-)-ethyl L-lactate and methyl acetate (1:3 v/v), and the reaction mixture was gently heated to 70 °C for 15 min. 15.4 g of (-)-(R)-camphor-10-sulfonic acid hydrate dissolved in the minimal amount of hot water was added into the reaction mixture. The reaction mixture was cooled gradually to 50-60 °C. The batch solution was maintained at 50-60 °C and seeded with clopidogrel (R)-camphorsulfonate, then cooled to 15 °C to effect crystallization of the product. The reaction mixture was further gradually cooled to 0 °C. The product was filtered, washed with methyl acetate, and then dried under vacuum at 25 °C. The diastereomeric salt was filtered, washed with acetone and dried. The yield was 20.1 g, having an optical purity of 99.8 % (+)-isomer (determined by chiral HPLC).

### Example 3

### Camphorsulfonate of the compound of formula (I)

5.0 g of a racemic mixture of the compound of formula (I) were dissolved in 15 mL of glyme and 3.5 mL ethoxyethanol at a temperature of 70 °C. 4.4 g of solid (-)-(R)-camphor-10-sulfonic acid were added into the solution. 20 mL of glyme were further added into the reaction mixture. The mixture was inoculated with 0.1 g of clopidogrel (R)-camphorsulfonate and allowed to cool down to room temperature. The mixture was stirred at room temperature until crystals began to form and then further cooled in a refrigerator. Crystals were recovered by filtration through sintered glass and washed with diethyl ether to obtain the camphorsulfonate of the compound of formula (I) having an optical purity of 99.9 % (+)-isomer (determined by chiral HPLC).

### Example 4

### Camphorsulfonate of the compound of formula (I)

5.0 g of an optically enriched mixture of the compound of formula (I) (85 % (+)-isomer) was dissolved in 6.5 mL of (-)-methyl L-lactate at a temperature of 70 °C. 2.2 g of solid (-)-(R)-camphor-10-sulfonic acid were added into the solution. 15 mL of diglyme were further added into the reaction mixture. The mixture was inoculated with 0.1 g of clopidogrel (R)-camphorsulfonate and allowed to cool down to room temperature. The mixture was stirred at room temperature until crystals began to form and then further cooled in a refrigerator. Crystals were recovered by filtration through sintered glass and washed with diethyl ether to obtain 2.1 g of the camphorsulfonate of the compound of formula (I), [α]_{D} = +23.95° (MeOH), having an optical purity of 99.7 % (+)-isomer.
The crystalline material thus obtained was dissolved in the minimal amount of the above solvent mixture necessary for dissolution at a temperature of 85-90 °C and re-crystallized. Crystals obtained after cooling to 0-5 °C were filtered off, washed with acetone and dried. The yield was 2.0 g, m.p. 165 °C, [α]_{D} = +24.74° (MeOH), optical purity practically 100 % (+)-isomer (determined by chiral HPLC).

### Example 5

### (-)-Dibenzoyl-L-tartrate of the compound of formula (I)

5.0 g of a racemic mixture of the compound of formula (I) were dissolved in 4 mL of (-)-ethyl L-lactate at a temperature of 75-80°C. 1.2 g of solid (-)-dibenzoyl-L-tartaric acid were added to the solution. 15 mL of toluene were slowly added to the resulting reaction mixture. The mixture was inoculated with 0.4 g of clopidogrel (R)-camphorsulfonate and allowed to cool down to room temperature. The mixture was stirred at room temperature until crystals began to form and then further cooled to 5 °C. Crystals were recovered by filtration through sintered glass and washed with diethyl ether to obtain 1.9 g of the (-)-dibenzoyl-L-tartrate of the compound of formula (I) having an optical purity of 99.7 % (+)-isomer (determined by chiral HPLC).

### Example 6

### Camphor sulfonate of the compound of formula (I)

Analogously to example 1, ethylene glycol monobutyl ether was used instead of n-butanol.
3.1 g of the (R)-camphorsulfonate of the compound of formula (I) were obtained in an optical purity of 99.9 % (+)-isomer (determined by chiral HPLC).

### Example 7

### Clopidogrel hydrogen sulfate

3.1 g of the camphorsulfonate of the compound of formula (I) obtained in example 1 were treated with a saturated aqueous solution of sodium bicarbonate (35 mL) in the presence of dichloromethane (40 mL). The organic phase was separated, washed with water (10 mL), dried with sodium sulfate and evaporated to dryness. Clopidogrel base was obtained as a slightly yellowish oil in quantitative yield, [α]_{D} = +51.53° (1.61 g/100 mL MeOH).

The residue was taken up in 30 mL of a mixture of acetone and butyl acetate (3.5:1 v/v), and an equimolar amount of concentrated sulfuric acid was added drop-wise at 5 °C under vigorous stirring. After further stirring at room temperature, the crystals formed were filtered off and washed with acetone to obtain 2.2 g of white crystals, m.p. 182°C.

### Example 8

### (S)-Camphorsulfonate of the compound of formula (I)

10.3 g of a racemic mixture of the compound of formula (I) were dissolved in 60 mL of ethylenglycol dimethyl ether at a temperature of 72-75 °C. 3.8 g of (+)-(S)-camphor-10-sulfonic acid were added to the solution in one portion with vigorous stirring. The mixture was stirred and allowed to gradually cool to room temperature. At a temperature 60°C, the mixture was inoculated with 0.3 g of the (S)-camphorsulfonate of the (R)-enantiomer of the compound of formula (I) and crystals began to form. The mixture was stirred at room temperature for 2 hours and then cooled in a refrigerator to a temperature of 0-5°C for 5 hours. Crystalline product was recovered by filtration, washed with a few drops of glyme, and 7.1 g of the (S)-camphorsulfonate of the compound of formula (I) were obtained, having an optical purity of 99.1 % (-)-isomer (determined by chiral HPLC).

### (R)-Camphorsulfonate of the compound of formula (I)

The combined filtrates were evaporated, and the resulting residue was dissolved in 50 mL of dichloromethane and 30 mL of a 10 % aqueous solution of NaHCO₃. The layers were separated, the dichloromethane layer was dried with MgSO₄, filtered, and the solvent was evaporated to obtain the free base of the compound of formula (I) as an oily residue. 5.5 g of said residue were dissolved in 27 mL of glyme and 5 mL of ethoxyethanol at a temperature of 72-75 °C. 3.4 g of (-)-(R)-camphor-10-sulfonic acid were added to the solution in one portion with vigorous stirring. The mixture was stirred and allowed to gradually cool to room temperature. At a temperature of 45-50 °C, the solution was inoculated with 0.15 g of clopidogrel (R)-camphorsulfonate and crystals began to form. The mixture was stirred at room temperature for 2 hours and then cooled in refrigerator to a temperature of 0-5 °C for 5 hours. Crystalline product was recovered by filtration, washed with a few drops of glyme, and 6.5 g of the (R)-camphorsulfonate of the compound of formula (I) were obtained, having an optical purity of 99.7 % (+)-isomer (determined by chiral HPLC).

The combined filtrates were evaporated at a temperature of 45-47 °C. The residue thus obtained was dissolved in 12 mL of glyme and 1.3 mL of (-)-ethyl L-lactate at a temperature of 72-75 °C. 0.85 g of (-)-(R)-camphor-10-sulfonic acid were added to the solution in one portion with vigorous stirring. The mixture was stirred and cooled down to room temperature. At a temperature of 45-50°C the solution was inoculated with 0.1 g of clopidogrel (R)-camphorsulfonate and crystals began to form. The mixture was stirred at room temperature for 2 hours and then cooled in a refrigerator to a temperature of 0-5 °C for 5 hours. Crystalline product was recovered by filtration, washed with a few drops of glyme, and 0.45 g of the (R)-camphorsulfonate of the compound of formula (I) were obtained in an optical purity of 100 % (+)-isomer (determined by chiral HPLC).

### Example 9

### Camphorsulfonate of the compound of formula (I) and racemization of undesired enantiomer of the compound of formula (I)

10.1 g of a racemic mixture of the compound of formula (I) were dissolved in 50 mL of glyme and 15 mL of (-)-ethyl L-lactate at a temperature of 72-75 °C. 4.4 g (-)-(R)-camphor-10-sulfonic acid were added to the solution in one portion with vigorous stirring. The mixture was stirred and cooled down to room temperature. At a temperature of 60 °C the solution was inoculated with 0.25 g of clopidogrel (R)-camphorsulfonate and crystals began to form. The mixture was stirred at room temperature for 2 hours and then cooled in a refrigerator to a temperature of 0-5°C for 5 hours. Crystalline product was recovered by filtration, washed with 2 mL of glyme, and 5.2 g of clopidogrel (R)-camphorsulfonate were obtained; having an optical purity of 99.7 % (+)-isomer.

### Regeneration of optically enriched compound of formula (I)

The combined filtrates were evaporated and the resulting residue was dissolved in 70 mL of dichloromethane and 35 mL of a 10 % aqueous solution of NaHCO₃. The layers were separated, the dichloromethane layer was dried with MgSO₄, filtered, and the solvent was evaporated (45°C/25 Torr). 7.1 g of optically enriched compound of formula (I) were obtained as an oily residue.

### Racemization of the compound of formula (I)

The product obtained immediately above was dissolved in 7.1 mL of glacial acetic acid and stirred at a temperature of 70-75°C for 2 hours (reaction progress was monitored by determination of optical rotation). When a sample of the reaction mixture gave [α] ∼ 0° (0.2 mL sample/5 mL MeOH, D line), the solvent was removed by distillation (70-75 °C/12-15 Torr) to yield 7.1 g of product having no acetic acid odor, (S):(R) = 50.1:49.9 (determined by chiral HPLC).

### Example 10

### Camphorsulfonate of the compound of formula (I)

7.1 g of the racemic mixture of the compound of formula (I) obtained in the last step of example 9 were dissolved in 39 mL of glyme at a temperature of 72-75 °C. 3.1 g of (-)-(R)-camphor-10-sulfonic acid were added to the solution in one portion with vigorous stirring. The mixture was stirred and cooled down to room temperature. At a temperature of 60 °C the solution was inoculated with clopidogrel (R)-camphorsulfonate and crystals began to form. The mixture was stirred at room temperature for 2 hours and then cooled in a refrigerator to a temperature of 0-5 °C for 5 hours. Crystalline product was recovered by filtration, washed with 2 mL of glyme, and 4.9 g of the (R)-camphorsulfonate of the compound of formula (I) was obtained, having an optical purity of (S):(R) = 99.2:0.8 (determined by chiral HPLC).

### Regeneration of optically enriched compound of formula (I)

The combined filtrates were evaporated, and the resulting residue was dissolved in 70 mL of dichloromethane and 35 mL of a 10 % aqueous solution of NaHCO₃. The layers were separated, the dichloromethane layer was dried with MgSO₄, decolorized with charcoal, filtered and the solvent was evaporated. 4.1 g of optically enriched compound of formula (I) were obtained as an oily residue.

### Racemization of the compound of formula (I)

3.7 g of the product obtained immediately above were dissolved in 3.7 mL of acetic acid and stirred at a temperature of 70-75 °C for 2 hours. When a sample of the reaction mixture gave [α] ∼ 0° (0.2 mL sample/5 mL MeOH, D line), the solvent was removed by distillation (70-75 °C/12-15 Torr) to yield 3.7 g of product, having no acetic acid odor, (S):(R) = 49.8:50.2 (determined by chiral HPLC).

The resolution, regeneration and racemization steps described above can be repeated several times in order to further improve the yield and quality of the product.

Similar results were obtained when the racemization step was performed using 3.7 mL of formic acid at a temperature of 40 - 50 °C or 3.7 mL of propionic acid at a temperature of 70 °C.

### Crystallization of the products obtained after several cycles

9.4 g of clopidogrel (R)-camphorsulfonate obtained after several of the above defined cycles of resolution, regeneration and racemization were dissolved in 8.5 mL of ethoxyethanol under gentle reflux. 25.5 mL of butyl acetate were added to the solution. The reaction mixture was inoculated with clopidogrel (R)-camphorsulfonate. Crystallization from the stirred solution started within 5-10 minutes. The mixture was cooled down under stirring, further stirred at 5 °C for 5 hours and then filtered under reduced pressure. The precipitate was washed with butyl acetate and dried under an infrared lamp to yield 9.2 g of the (R)-camphorsulfonate of the compound of formula (I), (S):(R) = 100:0 (determined by chiral HPLC).

### Example 11

### Clopidogrel 2-propyl sulfate

28.7 g of clopidogrel (R)-camphorsulfonate, prepared as described in example 2, were suspended in 163 mL of ethyl acetate, and 65 mL of water was added. The suspension was stirred at 20-25 °C and the pH was adjusted to 7.2 to 8 with slow addition of a 1 M aqueous solution of NaOH. The resulting mixture was stirred for 1 hour, and then the phases were allowed to separate. The organic layer was collected and thoroughly washed with 65 mL of water twice. The solvent was evaporated by vacuum distillation to obtain an oily residue. The residue was dissolved in 2-propanol (50 mL) and the solvent was again removed under vacuum to obtain 16.9 g of an oily residue. The exact amount of clopidogrel base (16.1 g) was determined by titration of an aliquot with HCl solution.

The residue was dissolved in 112 mL of 2-propanol. 2.9 mL of concentrated sulfuric acid were added and the reaction mixture was refluxed for about 2 hours. Then the mixture was inoculated with a few milligrams of clopidogrel 2-propyl sulfate while gradually being cooled to about 15 °C under stirring. The solid crystallized from the mixture was collected by filtration. Thus, 20.3 g of clopidogrel 2-propyl sulfate were obtained after drying under vacuum at 50 °C. Melting point (microscope according to Kofler): 158-163 °C, FT-IR spectrum (KBr, cm⁻¹): 1755, 1265, 1226, 1037, 935, 867, 618, ¹H-NMR (300 MHz, DMSO-d₆, TMS): δ (ppm) = 1.12 (d, J = 6.3 Hz, 6 H, 2x CH₃), 3. 6 - 4.2 (m, 6 H, 3x CH₂), 3.76 (m, 3 H, CH₃), 4.27 (m, J = 6.3 Hz, 1 H, CH), 5.59 (s, 1 H, CH), 6.89 (d, J = 5.4 Hz, thiophene), 7.44 (d, J = 5.4 Hz, thiophene), 7.5 -7.6 (m, 2 H, Ph), 7.65 - 7.72 (m, 2 H, Ph).

### Example 12

### Clopidogrel hydrochloride

28.7 g of clopidogrel (R)-camphorsulfonate, prepared as described in example 2, were suspended in 163 mL of ethyl acetate, and 65 mL of water was added. The suspension was stirred at 20-25 °C and the pH was adjusted to 7.2 to 7.7 with slow addition of an 8 wt.-% aqueous solution of NaHCO₃. The resulting mixture was stirred for 1 hour, and then the phases were allowed to separate. The organic layer was collected and thoroughly washed with 65 mL of water twice. The solvent was evaporated by vacuum distillation to obtain 16.6 g of an oily residue. The exact amount of clopidogrel base (16.2 g) was determined by titration of an aliquot with HCl solution. The residue was taken up in 110 mL of ethyl acetate, and the thus obtained solution was filtered and cooled to 0°C. 35.2 mL of a 1.5 M solution of HCl in ethyl acetate were added to the solution under stirring over about 0.5 hours. The resulting suspension was stirred with an overhead stirrer at a temperature of 0 °C for 2 hours, and then the temperature was raised to about 20 °C over about 3 hours. The suspension was further stirred for a few hours, and then the product was collected by filtration and washed with 22 mL of ethyl acetate. The product was dried under vacuum while gradually being heated to about 50 °C. 16.7 g of clopidogrel hydrochloride were obtained which exhibited XRPD peaks characteristic of polymorphic form I as described in WO 03/66637. The enantiomeric purity was more than 99.8 % (determined by chiral HPLC).

### Example 13

### Clopidogrel hydrogensulfate

28.7 g of clopidogrel (R)-camphorsulfonate, prepared as described in example 2, were suspended in 163 mL of methyl isobutyl ketone, and 65 mL of water were added. The suspension was stirred at 20-25 °C and the pH was adjusted to 7.0 to 7.6 with slow addition of an 8 wt.-% aqueous solution of NaHCO₃. The resulting mixture was stirred for 1 hour, and then the phases were allowed to separate. The organic layer was collected and thoroughly washed with 65 mL of water twice. The solvent was evaporated by vacuum distillation to obtain 17.0 g of an oily residue. The exact amount of clopidogrel base (16.3 g) was determined by titration of an aliquot with HCl solution. The residue was taken up in 326 mL of methyl isobutyl ketone, and the thus obtained solution was filtered and cooled to -10 to 0 °C. 2.62 mL of concentrated sulfuric acid were added to the solution under stirring over about 0.25 hours. The resulting suspension was stirred with an overhead stirrer at a temperature below 0 °C for 2 hours, then the temperature was raised to about 20 °C over about 5 hours. The suspension was further stirred for a few hours, and then the product was collected by filtration and washed with 22 mL of methyl isobutyl ketone. The product was dried under vacuum while gradually being heated to about 50 °C. 18.1 g of clopidogrel hydrogensulfate were obtained which exhibited XRPD peaks characteristic of polymorphic form I as described in WO 99/65915. The enantiomeric purity of the product was more than 99.8 % (determined by chiral HPLC).

## Claims

1. A process for the preparation of optically enriched clopidogrel of the following formula 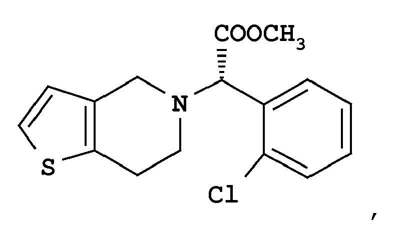 or a pharmaceutically acceptable salt or solvate thereof, comprising the steps of:
a) contacting a mixture comprising both enantiomers of the compound of formula (I) 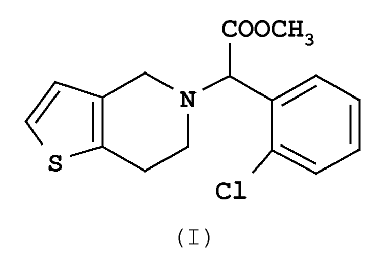 or a salt thereof with an optically active acid in a solvent or solvent mixture to give a mixture of diastereomeric acid addition salts,
wherein the solvent or solvent mixture comprises at least one solvent selected from the group consisting of optically active solvents, linear or branched (C₁-C₅)alkoxy(C₂-C₅) alcohols and linear or branched acyclic (C₁-C₅)alkoxy(C₂-C₅)alkyl (C₁-C₅)alkyl ethers;
b) separating the diasteromeric acid addition salts;
c) optionally reacting at least one of the separated acid addition salts with a base to give optically enriched clopidogrel; and
d) optionally converting the optically enriched clopidogrel into a pharmaceutically acceptable salt or solvate thereof.

2. The process according to claim 1, wherein the solvent or solvent mixture of step (a) comprises at least one optically active solvent.

3. The process according to claim 2, wherein the optically active solvent is an ester of L-lactic acid with a linear or branched (C₁-C₅)alcohol.

4. The process according to any one of claims 1 to 3, wherein the solvent or solvent mixture of step (a) comprises at least one linear or branched (C₁-C₅)alkoxy(C₁-C₅)alcohol.

5. The process according to any one of claims 1 to 4, wherein the solvent or solvent mixture of step (a) comprises at least one acyclic (C₁-C₅) alkoxy (C₂-C₅) alkyl (C₁-C₅) alkyl ether.

6. The process according to any one of claims 1 to 5, wherein the solvent mixture of step (a) comprises at least one linear or branched (C₁-C₅) alkoxy(C₂-C₅) alcohol in combination with at least one linear or branched acyclic alkoxy ether selected from (C₁-C₅)alkoxy(C₂-C₅)alkyl (C₁-C₅)alkyl ethers and bis ((C₁-C₅) alkoxy (C₂-C₅) alkyl) ethers, wherein (C₁-C₅)alkoxy(C₂-C₅)alkyl (C₁-C₅)alkyl ethers are preferred.

7. The process according to any one of claims 1 to 6, wherein the solvent mixture comprises optically active solvent in an amount of up to 50 % (v/v), preferably 2 - 30 % (v/v), most preferably 5 - 25 % (v/v) based on the total volume of solvents.

8. The process according to any of claims 1 to 7, wherein the weight ratio of optically active solvent to the compound of formula (I) is in the range of 0.5:1 to 50:1.

9. The process according to any one of claims 1 to 8, wherein the optically active acid used step (a) is selected from the group consisting of tartaric acid, mandelic acid, lactic acid, camphor sulfonic acid and bromocamphor sulfonic acid.

10. A process according to claim 9, wherein the optically active acid used in step (a) is (R)-camphor-10-sulfonic acid.

11. A process according to any one of claims 1 to 10, wherein the optically active acid used in step (a) is used in an amount of 0.4 to 1.2 equivalents with respect to the compound of formula (I).

12. A process according to any one of claims 1 to 10, wherein the separation of diastereomeric acid addition salts in step (b) is performed by crystallization from the reaction mixture obtained in step (a), optionally after addition of one or more additional solvent(s).

13. The process according to any one of claims 1 to 12, wherein the pharmaceutically active salt is clopidogrel hydrogensulfate.

14. The process according to any one of claim 13, wherein the pharmaceutically active salt is clopidogrel hydrogensulfate of crystalline form I.

15. A process for the preparation of a pharmaceutical formulation comprising substantially optically pure clopidogrel, or a pharmaceutically acceptable salt or solvate thereof, in combination with at least one pharmaceutically acceptable carrier, which includes the step of preparing the substantially optically pure clopidogrel or the pharmaceutically acceptable salt or solvate thereof by the process according to any one of claims 1 to 14.
